# EUROPÄISCHE PATENTSCHRIFT

(11) **EP 2 651 839 B1**
(45) Veröffentlichungstag und Bekanntmachung des Hinweises auf die Patenterteilung: **28.11.2018**
(21) Anmeldenummer: 11805477.4
(22) Anmeldetag: 19.12.2011
(51) Int. Cl.: C03C 3/091, C03C 11/00, C03C 4/00, C03B 19/06, A61K 6/027, A61C 13/00, A61C 13/08, A61K 6/033

(54) **HERSTELLUNG DENTALER FORMTEILE AUS PORÖSEM GLAS**
PRODUCTION OF SHAPED DENTAL PARTS COMPOSED OF POROUS GLASS
ÉLABORATION DE PIÈCES MOULÉES DENTAIRES EN VERRE POREUX

(30) Priorität: 17.12.2010 DE 102010056037
(43) Veröffentlichungstag der Anmeldung: 23.10.2013
(73) Patentinhaber: Ivoclar Vivadent AG, 9494 Schaan (LI)
(72) Erfinder: REINSHAGEN, Jörg, 75177 Pforzheim (DE); CRAMER VON CLAUSBRUCH, Sascha, 75417 Mühlacker-Lienzingen (DE); WINTERLING, Michael, 33142 Büren (DE)
(74) Vertreter: Uexküll & Stolberg
(86) Internationale Anmeldenummer: PCT/EP2011/073255
(87) Internationale Veröffentlichungsnummer: WO 2012/080513

(56) Entgegenhaltungen:
- EP-A1- 1 025 829
- EP-A1- 2 287 122
- EP-A1- 2 387 984
- EP-A2- 0 885 855
- DE-A1- 19 852 516
- US-A1- 2010 035 215

## Beschreibung

Die Erfindung betrifft die Verwendung eines Rohlings zur Herstellung dentaler Formteile sowie ein Verfahren zur Herstellung dieses Rohlings. Weiter betrifft die Erfindung ein Verfahren zur Herstellung monolithischer dentaler Formteile.

Zahnersatz aus Keramik bzw. Vollkeramik hat in den letzten Jahren eine immer größere Bedeutung erlangt. Bei dieser Art von Zahnersatz wird das keramische Material nicht nur, wie vorher bekannt, zur Verblendung von Gerüsten aus Metall eingesetzt, sondern auch das Gerüstmaterial selbst wird aus Keramik gefertigt. Auf diese Weise gelangt man zu vollkeramischem Zahnersatz, beispielsweise zu Vollkeramikkronen und Vollkeramikbrücken. In diesem Zusammenhang ist insbesondere Zirkoniumdioxid als keramisches Material hervorzuheben.

Bei vollkeramischem Zahnersatz werden die Gerüste, aber auch andere dentale Formteile wie Verblendungen, Abutments (Implantataufbauteile) oder sogar ganze Zähne, in der Regel aus einem Keramikblock, dem sogenannten Rohling (Blank), mechanisch herausgearbeitet, insbesondere herausgefräst. Solche Rohlinge können grundsätzlich aus (end-) gesintertem Keramikmaterial bestehen, das seine Dimensionen bei einer weiteren Wärmebehandlung nicht mehr ändert. Nachteilig ist hierbei jedoch, dass ein solches Material aufgrund seiner Härte nur schwer mechanisch bearbeitbar ist.

Deshalb hat sich die Verwendung von Rohlingen aus einem ungesinterten oder nicht (end-)gesintertem Keramikmaterial als vorteilhaft erwiesen. Solche Materialien können trocken mechanisch bearbeitet werden und sind beispielsweise in der WO-A1-2004/086999 offenbart. Eingesetzt werden diese Materialien in Verbindung mit einer CAD/CAM-Technik, die den Sinterschrumpf des Materials beim Endsintern bereits für das Herausarbeiten des Zahnersatzes aus dem Rohling berücksichtigt.

In der US 2010/0248189 A1 sind ein Verfahren zur Herstellung einer Verblendung für einen Zahnersatz sowie der Zahnersatz selbst beschrieben. Dabei wird ein Vorprodukt für die Verblendung aus einem Rohling hergestellt, wobei dieser Rohling oder dieses Vorprodukt aus einer porösen Glaskeramik oder einem porösen Glas bestehen. Die Offenbarung dieser Schrift bezieht sich dabei ausdrücklich auf Verblendungen, d.h. auf dentale Formteile, die zusammen mit einem weiteren Teil, in der Regel einem Gerüst zusammen den fertigen Zahnersatz bilden.

DE 198 52 516 A1 beschreibt ein Verfahren zur Herstellung einer keramischen Dentalrestauration auf Basis leucithaltiger Glaskeramik, bei dem ein Ausgangsglas oder eine Ausgangsglaskeramik in Form eines Pulvers oder Granulats verpresst und gesintert wird, um einen Sinterkörper in Zylinder- oder Pelletform zu erhalten, und der Sinterkörper im viskosen Zustand zu der keramischen Dentalrestauration verpresst wird.

Für die genannten Einsatzzwecke sind bereits unterschiedliche Materialien für die Bereitstellung von Rohlingen bekannt. Es besteht jedoch weiterer Bedarf an solchen Materialien, insbesondere an solchen, die trocken, d.h. ohne Kühlschmierstoffe, mechanisch bearbeitbar, insbesondere fräsbar sind.

Weiter ist nach Kenntnis der Anmelderin das Problem nicht gelöst, einen monolithischen Zahnersatz aus Glas aus einem trocken fräsbaren porösen Glasblank zu fertigen. Dies liegt unter anderem daran, dass Glas bei dem zur Reduzierung seiner Porosität erforderlichen Sinterschritt die Neigung hat, zu fließen. Damit verliert es jedoch als dentales Formteil seine Passung mit der Form des präparierten Zahnstumpfs, weshalb monolithische Zahnersatzteile aus Glas bisher kommerziell keine Anwendung gefunden haben.

Diese Aufgabe wird gelöst durch die Verwendung eines Rohlings zur Herstellung dentaler Formteile gemäß den Ansprüchen 1 bis 8, das Verfahren zur Herstellung eines Rohlings gemäß Anspruch 9 sowie das Verfahren zur Herstellung eines monolithischen dentalen Formteils gemäß den Ansprüchen 10 bis 16. Der Wortlaut sämtlicher Ansprüche wird hiermit durch Bezugnahme zum Inhalt dieser Beschreibung gemacht.

Der erfindungsgemäß verwendete Rohling zur Herstellung dentaler Formteile zeichnet sich dadurch aus, dass er vollständig aus porösem Glas ohne kristalline Anteile besteht. Dabei beträgt die Dichte dieses porösen Rohlings zwischen 50 % und 95 % seiner theoretischen Dichte (im dichtgesinterten Zustand). Weiter besitzt der Rohling eine scheibenartige Form mit einem Durchmesser von mindestens 20 mm.

Unter "Rohling" soll dabei jeder Körper der genannten Geometrie verstanden werden, aus dem dentale Formteile herausgearbeitet werden können, vorzugsweise durch eine mechanische Bearbeitung wie Fräsen, Schneiden u.dgl.. Solche Rohlinge werden häufig auch als "Blanks" bezeichnet.

Unter "scheibenartiger Form" soll erfindungsgemäß jede geometrische Ausgestaltung des erfindungsgemäß verwendeten Rohlings verstanden werden, bei der der entsprechende Körper eine deutlich geringere oder wesentlich geringere Dicke besitzt als der definierte Durchmesser von mindestens 20 mm. Der Begriff "scheibenartig" soll dabei zum Ausdruck bringen, dass die Erfindung nicht auf runde, d.h. im wesentlichen kreisförmige Scheiben beschränkt ist, sondern auch Abweichungen von der Kreisform einschließen soll. Dabei kann es sich beispielsweise um Rohlinge mit ovalen Querschnittsflächen, beispielsweise ellipsenförmigen Querschnittsflächen handeln. Auch andere Querschnittsflächen mit mindestens teilweise bogenförmigen Umfangslinien sollen umfasst sein, wie beispielsweise Scheiben, deren Außenumfang der äußeren Kontur eines Hufeisens nachgebildet ist.
Unter "Glas" ist ein amorpher, nicht kristalliner Feststoff zu verstehen. Es handelt sich um eine amorphe Substanz, die thermodynamisch als gefrorene, unterkühlte Flüssigkeit bezeichnet werden kann.

Bei den eingesetzten Gläsern handelt es sich vorzugsweise um oxidische Gläser und dabei insbesondere um Borosilikatgläser oder Alumosilikatgläser. Besonders geeignet für die vorliegende Erfindung sind Alkaliborosilikatgläser, da sich diese besonders gut als amorphe, poröse Festkörper darstellen lassen. Definitionsgemäß besitzen solche Alkaliborosilikatgläser als Bestandteile Alkalimetalloxide, in der Regel Natriumoxid (Na₂O) und/oder Kaliumoxid (K₂O) und Bortrioxid (B₂O₃). Weitere Bestandteile, in der Regel Hauptbestandteile, sind Siliziumdioxid (SiO₂) und Aluminiumoxid (Al₂O₃).
Unter "porös" soll bei der Erfindung verstanden werden, dass das Glas, aus dem der Rohling besteht, Poren, vorzugsweise mikroskopisch kleine Poren, besitzt. In der Regel sind die einzelnen Poren mindestens teilweise untereinander verbunden, so dass es sich hier um ein offenporiges System handelt. Die Porengröße bei porösen Gläsern lässt sich, insbesondere durch den Herstellungsprozess, innerhalb weiter Grenzen variieren, so dass Porengrößen zwischen 0,5 nm bis 5000 nm möglich sind. Auch die Porengrößenverteilung kann mehr oder weniger breit eingestellt werden.
Als "Dichte" soll bei der Erfindung in üblicher Weise das Gewicht des Glases (in Gramm g) pro cm³ verstanden werden. Die theoretische Dichte ist dabei derjenige Wert der Dichte, bei dem das Glas im wesentlichen porenfrei ist. Auf den Rohling bezogen, ist das der Zustand, in dem der Rohling dichtgesintert und damit weitgehend porenfrei ist.

In Weiterbildung beträgt die Dichte des erfindungsgemäß verwendeten Rohlings vorzugsweise zwischen 55 % und 85 %, insbesondere zwischen 70 % und 80 %, der theoretischen Dichte des Rohlings.

Wie bereits erläutert können bei der Erfindung insbesondere Silikatgläser wie Borosilikatgläser, vorzugsweise Alkaliborosilikatgläser, eingesetzt werden. Derartige Gläser können mit Vorteil aus den folgenden Komponenten zusammengesetzt sein:

| | |
|---|---|
| SiO₂ | 50 - 80 Gew.-% |
| Al₂O₃ | 3 - 24 Gew.-% |
| K₂O | 3 - 13 Gew.-% |
| Na₂O | 3 - 13 Gew.-% |
| Li₂O | 0 - 4 Gew.-% |
| B₂O₃ | 0 - 4 Gew.-% |
| F | 0 - 4 Gew.-% |
| TiO₂ | 0 - 8 Gew.-% |
| ZrO₂ | 0 - 8 Gew.-% |
| P₂O₅ | 0 - 4 Gew.-% |
| SnO₂ | 0 - 4 Gew.-% |
| MgO | 0 - 4 Gew.-% |
| CaO | 0 - 4 Gew.-% |
| BaO | 0 - 4 Gew.-% |
| Sb₂O₃ | 0 - 4 Gew.-% |
| CeO₂ | 0 - 8 Gew.-%. |

Weiter bevorzugt sind bei der Erfindung Rohlinge, bei denen das verwendete poröse Glas entweder aus den Komponenten

| | |
|---|---|
| SiO₂ | 55 - 65 Gew.-% |
| Al₂O₃ | 17 - 24 Gew.-% |
| K₂O | 5 - 9 Gew.-% |
| Na₂O | 7 - 11 Gew.-% |
| Li₂O | 0 - 1 Gew.-% |
| B₂O₃ | 0 - 2 Gew.-% |
| F | 0 - 1 Gew.-% |
| TiO₂ | 0 - 1 Gew.-% |
| ZrO₂ | 0 - 2 Gew.-% |
| P₂O₅ | 0 - 1 Gew.-% |
| SnO₂ | 0 - 1 Gew.-% |

oder den Komponenten

| | |
|---|---|
| SiO₂ | 55 - 80 Gew.-% |
| Al₂O₃ | 7 - 23 Gew.-% |
| K₂O | 3 - 10 Gew.-% |
| Na₂O | 3 - 13 Gew.-% |
| Li₂O | 0 - 1 Gew.-% |
| B₂O₃ | 0 - 4 Gew.-% |
| F | 0 - 1 Gew.-% |
| TiO₂ | 0 - 1 Gew.-% |
| ZrO₂ | 0 - 8 Gew.-% |
| P₂O₅ | 0 - 1 Gew.-%. |

zusammengesetzt ist.

Insbesondere besitzt der erfindungsgemäß verwendete Rohling die Form einer im wesentlichen kreisrunden Scheibe mit einem Durchmesser von mindestens 20 mm. Dabei sind Durchmesser von mindestens 50 mm noch weiter bevorzugt. Auch Durchmesser von mindestens 80 mm sind mit Vorteil möglich.

Bei allen scheibenartigen Rohlingen, insbesondere bei den zuletzt genannten Scheiben, die im wesentlichen kreisrund sind, beträgt die Dicke der Scheibe insbesondere mehr als 5 mm. Dabei sind Dicken der Scheiben zwischen 5 mm und 30 mm noch weiter bevorzugt.

Die erfindungsgemäß verwendeten Rohlinge können die Farbe aufweisen, die durch das zu seiner Herstellung verwendete poröse Glas vorgegeben ist. Mit Vorteil können die Rohlinge jedoch auch eingefärbt sein. Dies kann durch die Zugabe von Zusatzstoffen zum Glas geschehen, die die Färbung des Glases beeinflussen.

So können bereits dem Glas bei dessen Herstellung oder dem Rohling bei seiner Herstellung, färbende Zusätze, insbesondere Metalloxide und andere Metallsalze zugesetzt werden. Bei den entsprechenden Metallen kann es sich beispielsweise um die Seltenerdenelemente oder die Nebengruppenelemente des Periodensystems der Elemente handeln. Vorzugsweise handelt es sich bei diesen Metallen um Eisen, Chrom, Aluminium, Zinn, Zink, Vanadium, Selen, Silber, Indium, Neodym, Samarium, Europium, Praseodym, Kobalt, Nickel, Mangan, Erbium oder Cer.
Dabei ist es auch möglich, die Rohlinge nach ihrer Herstellung einzufärben, beispielsweise unter Verwendung von Färbelösungen, die die genannten Elemente in Form von Ionen oder Komplexionen enthalten.

Weiter können zur Einfärbung des Glases auch die sogenannten keramischen Pigmente als färbende Zusätze verwendet werden. Derartige Pigmente sind dem Fachmann ebenfalls bekannt. Hierbei handelt es sich um Oxide und Silikate mit kristalliner Struktur, beispielsweise vom Typ Spinell, Zirkon u.a.. Es gibt dabei zum einen die Pigmente, bei denen die Kristalle eine Eigenfarbe besitzen, und zum anderen die sogenannten Einschlusspigmente, bei denen farblose Kristalle farbige Einschlüsse aufweisen.

Bei den genannten eingefärbten Rohlingen nach der Erfindung kann der gesamte Rohling einheitlich eingefärbt sein. Es ist jedoch auch möglich, einzelne Teile des Rohlings oder den gesamten Rohling unterschiedlich einzufärben und dabei beispielsweise einen kontinuierlichen Farbverlauf innerhalb des Rohlings zu erzeugen.
In Weiterbildung kann der erfindungsgemäß verwendete Rohling mindestens ein Haltemittel zum Einspannen des Rohlings bei seiner Bearbeitung zu einem dentalen Formteil aufweisen. Dieses Haltemittel dient somit zur (in der Regel reversiblen) Halterung oder Befestigung des Rohlings in einer entsprechenden Bearbeitungsmaschine, beispielsweise einer Fräsmaschine.
Vorzugsweise ist das mindestens eine Haltemittel direkt am Rohling angeformt, beispielsweise auch durch eine Befestigung, wie eine Verklebung, oder ausgeformt, wie beispielsweise durch mindestens eine, mindestens teilweise am Außenumfang des Rohlings umlaufende Ausnehmung, insbesondere Nut.
Neben der Verwendung des beschriebenen Rohlings umfasst die Erfindung auch ein Verfahren zur Herstellung eines so verwendeten Rohlings. Dieses Verfahren ist dadurch gekennzeichnet, dass Glaspulver bei einem Druck zwischen 10 MPa und 300 MPa zu einem Grünkörper verpresst und dieser Grünkörper dann bei einer Temperatur zwischen 580 °C und 750 °C zu einem Rohling aus porösem Glas (ohne kristalline Anteile) gesintert (vorgesintert) wird.
Der genannte angewendete Druck beim Verpressen beträgt dabei vorzugsweise zwischen 50 MPa und 250 MPa, insbesondere zwischen 100 MPa und 200 MPa.

Die Temperatur beim Vorsintern beträgt dabei vorzugsweise zwischen 580 °C und 700 °C, insbesondere zwischen 620 °C und 660 °C.

Die Dauer der Temperaturbehandlung beim Vorsintern, d.h. die sogenannten Haltezeiten liegen zwischen 0,5 h und 10 h, insbesondere zwischen 1 h und 5 h. Innerhalb des zuletzt genannten Bereichs sind Haltezeiten zwischen 1 h und 3 h weiter bevorzugt.

Schließlich soll hervorgehoben werden, dass das Vorsintern unter reduziertem Atmosphärendruck oder unter Vakuum erfolgen kann.

Bezüglich des Pressvorganges können alle Methoden angewendet werden, die für das Pressen derartiger Glaspulver einsetzbar sind. Vorzugsweise wird das Glaspulver dabei trocken gepresst. Möglich ist ein uniaxiales Pressen, d.h. ein Aufbringen des Pressdrucks in axialer Richtung von einer Seite, oder ein biaxiales Pressen, d.h. ein Aufbringen des Pressdrucks in axialer Richtung sowohl von der einen als auch von der anderen Seite.
Auch ein isostatisches Pressen, d.h. ein Aufbringen des Pressdrucks aus allen Richtungen, oder ein quasiisostatisches Pressen, d.h. eine Kombination von axialem Pressen und Pressen am Außenumfang, ist möglich.

Weiter umfasst die Erfindung ein Verfahren zur Herstellung eines monolithischen dentalen Formteils gemäß Anspruch 10. Dieses Verfahren ist dadurch gekennzeichnet, dass ein erfindungsgemäß verwendeter Rohling mechanisch bearbeitet (unter Ausbildung eines monolithischen Formteils) wird und das so erhaltene Formteil auf mindestens 98 % seiner theoretischen Dichte gesintert (insbesondere endgesintert) wird. Die mechanische Bearbeitung erfolgt dabei vorzugsweise durch mindestens einen Fräsvorgang. Weiter handelt es sich bei der mechanischen Bearbeitung insbesondere um eine trockene mechanische Bearbeitung, d.h. um eine Bearbeitung ohne Verwendung von Kühlmitteln, wie Kühlflüssigkeiten.

Unter "monolithisch" soll dabei jedes aus nur einem Teil bestehende dentale Formteil verstanden werden, das für die entsprechende Anwendung einen vollständigen Zahnersatz bildet. Dementsprechend bedeutet monolitisch auch einheitlich oder einstückig. Diese Definition bringt den Unterschied zu einem zwei- oder mehrteiligen Zahnersatz zum Ausdruck, der beispielsweise aus einem Gerüst und einer auf dieses Gerüst aufgesetzten Verblendung besteht.

Dementsprechend kann es sich bei den erfindungsgemäß hergestellten monolithischen dentalen Formteilen bzw. dem monolithischen Zahnersatz um (aus einem Stück bestehende) Kronen, Brücken, Inlays, Onlays oder dergleichen handeln. Alle diese Teile können vom Zahnarzt direkt in den Mund des Patienten eingesetzt werden.

Erfindungsgemäß ist das Verfahren dadurch gekennzeichnet, dass das monolithische Formteil während mindestens eines Zeitabschnitts innerhalb der Sinterdauer mit mindestens einer Stützkonstruktion oder mit mindestens einer Stützform in Verbindung gebracht wird und zwar derart, dass die während dieser Sinterdauer eintretende Formänderung des Formteils durch die Stützkonstruktion oder die Stützform (räumlich) begrenzt wird.
Mit anderen Worten: Die Stützkonstruktion oder die Stützform verhindert, dass die beim Sintervorgang auftretende Formänderung des Dentalteils (Sinterschrumpf) über das durch die Stützkonstruktion/Stützform vorgegebene Maß hinausgeht.

Vorzugsweise ist das beschriebene Verfahren so ausgestaltet, dass das Formteil zumindest während eines Zeitabschnitts am Ende der gesamten Sinterdauer mit der Stützkonstruktion/Stützform in Verbindung gebracht wird. Damit wird erreicht, dass die Begrenzung der Formänderung des Dentalteils durch die Stützkonstruktion/Stützform zumindest am Ende des Sintervorgangs gewährleistet ist. Man kann also in solchen Fällen über einen ersten, ggf. längeren, Zeitraum ohne Stützkonstruktion/Stützform sintern und damit eine (nicht beeinflußte) Formänderung des Dentalteils in Kauf nehmen, und dann während eines zweiten, ggf. kürzeren, Zeitabschnitts am Ende des Sintervorgangs mit Stützkonstruktion/Stützform sintern. Dann wird die Formänderung, d.h. der Sinterschrumpf, wie oben erläutert, am Ende des Sintervorgangs in der erwünschten Weise begrenzt.
In diesem Zusammenhang ist es auch möglich, während eines Gesamtsintervorgangs mit mehreren Stützkonstruktionen/Stützformen unterschiedlicher Geometrie bzw. unterschiedlicher Abmessungen zu arbeiten. Man kann in diesen Fällen dann die Formänderung des Dentalteils während eines ersten Zeitabschnitts durch eine erste Stützkonstruktion/Stützform begrenzen und dann in einem nächsten Zeitabschnitt durch eine zweite Stützkonstruktion/Stützform mit anderen Abmessungen (usw.). Auf diese Weise wird das Dentalteil während des Sintervorgangs schrittweise an seine endgültige Form (nach dem Sintern) angenähert.
Dieses schrittweise Vorgehen kann auch so ablaufen, dass einzelne Teile oder Abschnitte des Formteils durch Stützkonstruktionen/Stützformen unterschiedlicher Geometrie unter Begrenzung der Formänderung für diesen Teil/diesen Abschnitt nacheinander in die gewünschte Endform gebracht werden.

Je nach Vorgehensweise bei dem beschriebenen Verfahren gemäß Anspruch 10 kann es bevorzugt sein, dass die Stützkonstruktion oder die Stützform aus einem temperaturbeständigen Material besteht, das selbst seine Form während des Sinterns nicht oder nur wenig ändert. Vorzugsweise beträgt die Formänderung des temperaturbeständigen Materials in solchen Fällen weniger als 2 Vol.-%, insbesondere weniger als 1 Vol.-%.

In Weiterbildung kann das erfindungsgemäße Verfahren gemäß Anspruch 10 auch so ausgestaltet sein, dass die Stützkonstruktion oder die Stützform aus einem Material besteht, dessen Formänderung während des Sinterns, d.h. dessen Sinterschrumpf, der Formänderung des dentalen Formteils beim Sintern, d.h. dessen Sinterschrumpf, entspricht. Dadurch ändern Stützkonstruktionen/Stützform einerseits und dentales Formteil andererseits ihre Form während des Sinterns in gleicher Weise, so dass beispielsweise ein Formschluss zwischen Stützkonstruktion/Stützform und dentalem Formteil zu Beginn des Sinterns während des Sintervorgangs bis zum Ende des Sinterns erhalten bleibt. Ggf. kann in solchen Fällen die Formänderung des dentalen Formteils beim Sintern auch durch die Formänderung der Stützkonstruktion/Stützform während des Sinterns gesteuert werden, beispielsweise dadurch, dass man für das Material der Stützkonstruktion/Stützform bewusst ein anderes Schrumpfverhalten auswählt als das Schrumpfverhalten, das das dentale Formteil zeigt.

Die genannten temperaturbeständigen Materialien für die Herstellung der Stützkonstruktion oder der Stützform sind in der Dentaltechnik als sogenannte Feuerfestmaterialien bekannt. Sie werden in der Regel als sogenannte Einbettmassen eingesetzt. Diese bestehen in der Regel aus drei wesentlichen Komponenten, nämlich einer feuerfesten Grundmasse, einem vorzugsweise anorganischen Bindemittel und Zusatzstoffen. Bei den Grundmassen handelt es sich typischerweise um Materialien auf Basis von SiO₂ (Quarz und/oder Cristobalit). Bindemittel sind beispielsweise wässrige Dispersionen von Ethylsilikat oder Natriumsilikat (Wasserglas).

Zur Anwendung kommen in der Regel phosphatgebundene, CalciumSulfat gebundene und Quarz gebundene Einbettmassen. Diese Massen werden auch als Spezialgipse bezeichnet. Je nach Anteil der einzelnen Komponenten bzw. der Zusammensetzung der Einbettmasse ergeben sich eine individuell einstellbare Expansion/Schwindung der Masse beim Sintern und die maximale Temperaturbeständigkeit der Masse. Bevorzugt sind Einbettmassen/Feuerfestmassen mit geringer Expansion (< 3%) bzw. mit Null-Expansion und Massen mit einer Temperaturbeständigkeit von größer 900°C bzw. größer 1000°C.

Geeignete Stützkonstruktionen/Stützformen können aus solchen Materialien beispielsweise durch mechanische Bearbeitung wie Fräsen oder mit Hilfe von Gießverfahren hergestellt werden.

Je nach dem, welches monolithische Formteil beim Sinterschritt stabilisiert werden soll, kann die Stützkonstruktion/Stützform unterschiedlich ausgestaltet sein. Handelt es sich beispielsweise um eine monolithische Krone, so liegt die Stützkonstruktion/Stützform als Positivform vor, vorzugsweise in der Form eines Zahnstumpfes, auf den die Krone zumindest während eines Teils des Sintervorganges aufgesetzt wird. Dadurch ist die Formstabilisierung zwischen der Außenfläche der Stützkonstruktion/Stützform, hier in Form eines Zahnstumpfes, und der Innenfläche der Krone gewährleistet. Die Stützkonstruktion/Stützform in der Form des Zahnstumpfes kann dabei beispielsweise durch Dublieren des Modells, das anhand des Zahnabdrucks gewonnen wurde, oder auch durch Fräsen anhand digitalisierter Daten des Modells erhalten werden.

Handelt es sich bei dem monolithischen Formteil beispielsweise um ein sogenanntes Inlay, so erfolgt die Formstabilisierung dadurch, dass die Stützkonstruktion/Stützform eine Negativform für das dentale Formteil bildet. Dementsprechend stabilisiert hier die Innenfläche der Stützkonstruktion/Stützform die Außenfläche des dentalen Formteils, zumindest während eines Teils des Sintervorgangs.

Die Temperaturbehandlung beim Endsintern erfolgt vorzugsweise über einen Zeitraum (Haltezeiten) zwischen 0,5 min (30 s) und 1 h, insbesondere zwischen 0,5 min und 20 min. Innerhalb der zuletzt genannten Zeiträume sind Haltezeiten zwischen 0,5 min und 10 min, insbesondere zwischen 1 min und 5 min, weiter bevorzugt.

Der Sinterschwund des hergestellten dentalen Formteils während des Endsinterns liegt dabei in der Regel unterhalb von 20 % (Volumenverringerung), vorzugsweise zwischen 5 % und 15 %.

Die Temperaturbehandlung beim Endsintern kann dabei mit Vorteil ebenfalls unter reduziertem Atmosphärendruck, vorzugsweise unter Vakuum, erfolgen.

Mit Vorteil wird das erfindungsgemäße Verfahren zur Herstellung der dentalen Formteile so ausgestaltet, dass das monolithische Formteil nach dem Endsintern immer noch aus Glas, welches im wesentlichen keine kristallinen Anteile besitzt, besteht. Dies geschieht bei den entsprechend zusammengesetzten Gläsern insbesondere durch die Ausgestaltung der Temperaturbehandlung (Höhe der Sintertemperatur, Haltezeit). Dementsprechend wird durch das Endsintern nur die Porosität des Glases, d.h. die Porenzahl im Glas, reduziert, ohne dass durch die Temperaturbehandlung kristalline Anteile im Glas entstehen.

Es ist jedoch auch möglich, insbesondere durch den Verfahrensschritt des Endsinterns und/oder durch die Auswahl der Bestandteile des verwendeten Glases, beim Endsintern kristalline Anteile im Glas zu erzeugen. In diesen Fällen besteht dann das monolithische dentale Formteil (im Gegensatz zu dem für seine Herstellung verwendeten Rohling) nach seiner Fertigstellung nicht mehr oder nicht mehr ausschließlich aus amorphem Glas. Es handelt sich also um eine Glaskeramik, welche in der Regel in einer amorphen (Glas-)Matrix kristalline (keramische) Bereiche aufweist.

Die Sintertemperaturen beim Endsintern, die während der als bevorzugt genannten Haltezeiten eingehalten werden, liegen bei dem erfindungsgemäßen Herstellungsverfahren für die dentalen Formteile insbesondere zwischen 800 °C und 1100 °C. Dabei sind Sintertemperaturen zwischen 900 °C und 1050 °C weiter bevorzugt.

Auch bei dem Herstellungsverfahren für die dentalen Formteile ist es möglich, diese Formteile einzufärben. Dies geschieht dann vorzugsweise nach der mechanischen Bearbeitung vor dem Endsintern, insbesondere mit Hilfe der oben genannten Färbelösungen.

Vorzugsweise handelt es sich bei den durch das beschriebene Verfahren erhaltenen monolitischen dentalen Formteilen um Kronen, Teilkronen oder Brücken sowie um Inlays, Onlays oder um ganze Zähne.

Der sogenannte WAK-Wert (Wärmeausdehnungskoeffizient) (gemessen nach ISO-6872) beträgt bei den erfindungsgemäß erhaltenen dentalen Formteilen vorzugsweise weniger als 10 x 10⁻⁶ 1/K und liegt dabei insbesondere zwischen 7 und 10 x 10⁻⁶ 1/K.

Weitere Merkmale der Erfindung ergeben sich aus den nachfolgenden Beispielen in Verbindung mit den Unteransprüchen. Hierbei können die einzelnen Merkmale jeweils für sich alleine oder zu mehreren in Kombination miteinander bei einer Ausführungsform der Erfindung verwirklicht sein.

In den Zeichnungen zeigen:
- Fig. 1: die Darstellung eines Röntgendiffraktogramms von erfindungsgemäß verwendeten Rohlingen,
- Fig. 2: die schematische Darstellung einer Ausführungsform des erfindungsgemäßen Verfahrens zur Herstellung monolithischer dentaler Formteile.

### Beispiel 1

Ein erstes Glaspulver mit der chemischen Zusammensetzung entsprechend Tabelle 2 und der Partikel- bzw. Korngrößenverteilung entsprechend Tabelle 3 wurde mittels eines hydraulischen Pressautomaten zu kreisrunden scheibenförmigen Rohlingen (Grünkörpern) mit einem Durchmesser von ca. 100 mm und einer Dicke von 18 mm uniaxial verpresst. Die Presskraft betrug zwischen 800 kN und 2000 kN, bevorzugt ca. 1200 kN.

Bei der Herstellung solcher scheibenförmiger Rohlinge ist es von Vorteil, wenn das zur Herstellung verwendete Glaspulver mit einem Binder granuliert wird. Dies wird in der Regel durch eine sogenannte Sprühgranulierung erreicht. Hierbei wird das Glaspulver unter gleichzeitiger Vermischung mit dem Binder zu einem Granulat verarbeitet. Derartige Verfahren sind dem Fachmann ohne Weiteres bekannt.

Drei Gruppen von Rohlingen wurden anschließend in Luftatmosphäre bei 640 °C, bei 660 °C bzw. bei 680° C während einer Haltezeit von 2 Stunden angesintert (Tabelle 1). Die bevorzugte Ansintertemperatur betrug 640 °C. Höhere Temperaturen führten zu zunehmend harten Rohlingen, die nur schwierig (hoher Werkzeugverschleiß) zu fräsen waren. Unter 640 °C wurden die Rohlinge zunehmend weich, so dass die Rohlinge vorsichtig bearbeitet und behandelt werden mussten. Die Dichte der Rohlinge betrug nach dem Ansintern bei 640 °C 1,79 g/cm³, bei 660 °C 1,82 g/cm³ und bei 680 °C 1,87 g/cm³.

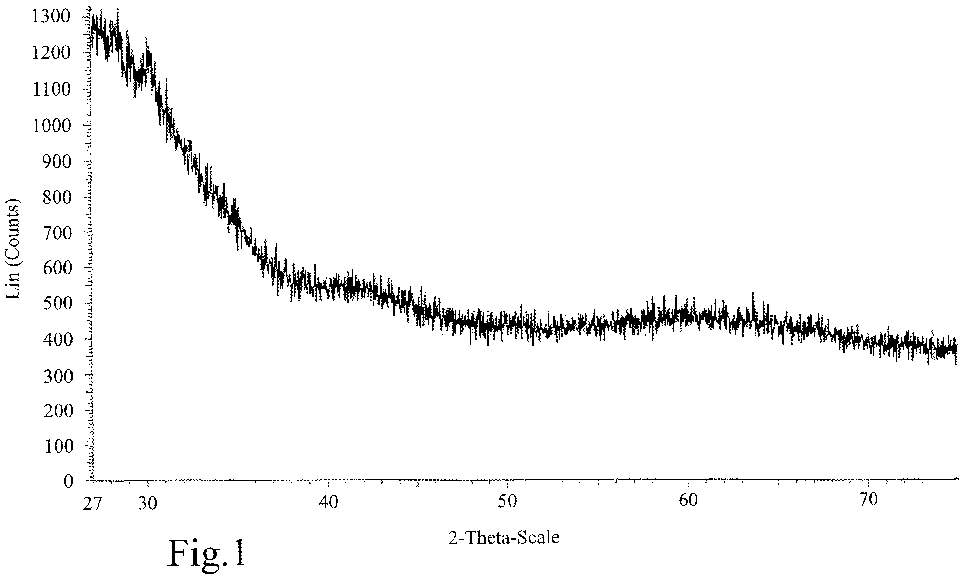

Wie in der Beschreibung erläutert, besteht der so hergestellte Rohling aus porösem Glas ohne kristalline Anteile. Dies zeigt für die Rohlinge, die gemäß Beispiel 1 hergestellt wurden, Fig. 1. Bei Fig. 1 handelt es sich um ein Röntgendiffraktogramm, das an den gemäß Beispiel 1 verpressten und angesinterten Rohlingen ermittelt wurde. In üblicher Weise ist dabei die Intensität gegen den Winkel aufgetragen. Fig. 1 zeigt keinerlei Beugungs-Intensitätsmaxima, die für kristalline Feststoffe oder kristalline Anteile in solchen Feststoffen charakteristisch wären. Es zeigt sich nur die bekannte diffuse Streuung, die für amorphe Stoffe charakteristisch ist.
Dementsprechend handelt es sich bei den gemäß Beispiel 1 hergestellten Rohlingen um amorphe Festkörper ohne kristalline Anteile.

Die bei 640 °C angesinterten Rohlinge wurden in ein Halteelement gespannt und mittels CAD/CAM Fräsbearbeitung unter Berücksichtigung des Sinterschwunds bearbeitet. Es wurden so aus den Rohlingen um den Sinterschwund korrigierte, vergrößerte Kronen als monolithische Formteile gefräst. Die Ergebnisse sind in Tabelle 1 dargestellt.

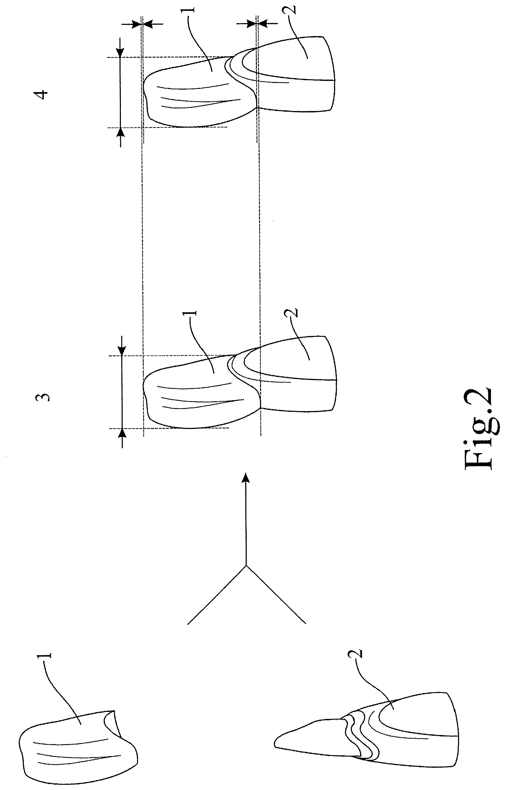

Wie in Fig. 2 schematisch dargestellt ist, wurden die so gefrästen (um den Sinterschwund vergrößerten) Kronen 1 auf einen Modellstumpf 2 aus nicht schwindendem Feuerfestmaterial (Einbettmasse Wilavest quick der Anmelderin) aufgesetzt und im Dentalofen auf den Stumpf formschlüssig aufgesintert. In Fig. 2 zeigt Anordnung 3 Stumpf 2 mit aufgesetzter Krone 1 vor dem Sintern und Anordnung 4 Stumpf 2 mit aufgesetzter Krone 1 nach dem Sintern. Der beim Sintern eintretende Sinterschrumpf, der im vorliegenden Fall ca. 10 % beträgt, ist bei Anordnung 4 durch die Pfeildarstellungen (rechts) skizziert.

Zur leichten Entfernung der Krone wurde der Stumpf aus Feuerfestmaterial mit einem Hochtemperaturtrennmittel (BN-Pulver, mit Pinsel aufgetragen; alternativ auch als Spray) dünn beschichtet. Ein solches Trennmittel ist aber nicht zwingend erforderlich. Der Modellstumpf selbst wurde durch Abgießen der Feuerfestmasse in so genannte Dublierformen oder durch Fräsen aus dem entsprechenden Material hergestellt. Nach der Sinterung wurden transparente und zahnfarbene gesinterte Kronen erhalten, die sich der Stumpfkontur angepasst hatten und die leicht vom Stumpfmaterial getrennt werden konnten.

Die WAK-Werte der Formteile betragen 9,3 ± 0,5 x 10⁻⁶ 1/K (25 °C bis 500 °C).

**Tabelle 1: Hergestellte Glasblanks aus dem ersten Glaspulver**

| Nr. | Pressgeometrie [mm] | Pressdruck [kN] | Ansintertemperatur [°C] | Dichte [g/cm³] |
|---|---|---|---|---|
| 1 | 100 mm x 18 mm | 1200 | 640 | 1,79 |
| 2 | 100 mm x 18 mm | 1200 | 660 | 1,82 |
| 3 | 100 mm x 18 mm | 1200 | 680 | 1,87 |

**Tabelle 2: Chemische Zusammensetzung des ersten Glaspulvers**

| Stoff | Anteil [Gew.-%] |
|---|---|
| SiO₂ | 60,5 |
| Al₂O₃ | 22,0 |
| K₂O | 8,0 |
| Na₂O | 9,0 |
| B₂O₃ | 0,5 |

Die theoretische Dichte dieses Glaspulvers beträgt ca. 2,45 g/cm³.

**Tabelle 3: Partikelgrößenverteilung des ersten Glaspulvers**

| Verteilung | Durchmesser [µm] |
|---|---|
| d 10 | 1,5 |
| d 50 | 9 |
| d 90 | 45 |

d 10, d 50, und d 90 bedeutet, dass 10 %, 50 % bzw. 90 % der vorhandenen Partikel kleiner als der angegebene Wert für den Durchmesser sind.

## Patentansprüche

1. Verwendung eines Rohlings, der vollständig aus porösem Glas ohne kristalline Anteile besteht, wobei die Dichte dieses porösen Rohlings zwischen 50 % und 95 % der theoretischen Dichte eines dichtgesinterten Rohlings beträgt, und der eine scheibenartige Form mit einem Durchmesser von mindestens 20 mm aufweist, zur Herstellung dentaler Formteile.

2. Verwendung nach Anspruch 1, bei der die Dichte des Rohlings zwischen 55 % und 85 %, vorzugsweise zwischen 70 % und 80 %, der theoretischen Dichte des Rohlings beträgt.

3. Verwendung nach Anspruch 1 oder 2, bei der das Glas aus den folgenden Komponenten zusammengesetzt ist:
| | |
|---|---|
| SiO₂ | 50 - 80 Gew.-% |
| Al₂O₃ | 3 - 24 Gew.-% |
| K₂O | 3 - 13 Gew.-% |
| Na₂O | 3 - 13 Gew.-% |
| Li₂O | 0 - 4 Gew.-% |
| B₂O₃ | 0 - 4 Gew.-% |
| F | 0 - 4 Gew.-% |
| TiO₂ | 0 - 8 Gew.-% |
| ZrO₂ | 0 - 8 Gew.-% |
| P₂O₅ | 0 - 4 Gew.-% |
| SnO₂ | 0 - 4 Gew.-% |
| MgO | 0 - 4 Gew.-% |
| CaO | 0 - 4 Gew.-% |
| BaO | 0 - 4 Gew.-% |
| Sb₂O₃ | 0 - 4 Gew.-% |
| CeO₂ | 0 - 8 Gew.-% |
und gegebenenfalls färbende Zusätze.

4. Verwendung nach Anspruch 3, bei der das Glas die folgende Zusammensetzung aufweist:
| | |
|---|---|
| SiO₂ | 55 - 65 Gew.-% |
| Al₂O₃ | 17 - 24 Gew.-% |
| K₂O | 5 - 9 Gew.-% |
| Na₂O | 7 - 11 Gew.-% |
| Li₂O | 0 - 1 Gew.-% |
| B₂O₃ | 0 - 2 Gew.-% |
| F | 0 - 1 Gew.-% |
| TiO₂ | 0 - 1 Gew.-% |
| ZrO₂ | 0 - 2 Gew.-% |
| P₂O₅ | 0 - 1 Gew.-% |
| SnO₂ | 0 - 1 Gew.-% |
und gegebenenfalls färbende Zusätze.

5. Verwendung nach Anspruch 3, bei der das Glas die folgende Zusammensetzung aufweist:
| | |
|---|---|
| SiO₂ | 55 - 80 Gew.-% |
| Al₂O₃ | 7 - 23 Gew.-% |
| K₂O | 3 - 10 Gew.-% |
| Na₂O | 3 - 13 Gew.-% |
| Li₂O | 0 - 1 Gew.-% |
| B₂O₃ | 0 - 4 Gew.-% |
| F | 0 - 1 Gew.-% |
| TiO₂ | 0 - 1 Gew.-% |
| ZrO₂ | 0 - 8 Gew.-% |
| P₂O₅ | 0 - 1 Gew.-% |
und gegebenenfalls färbende Zusätze.

6. Verwendung nach einem der vorhergehenden Ansprüche, bei der der Rohling die Form einer im Wesentlichen kreisrunden Scheibe mit einem Durchmesser von mindestens 20 mm, insbesondere von mindestens 50 mm, aufweist, wobei Durchmesser von mindestens 80 mm weiter bevorzugt sind.

7. Verwendung nach einem der vorhergehenden Ansprüche, bei der die Dicke der Scheibe mehr als 5 mm, vorzugsweise zwischen 5 mm und 30 mm beträgt.

8. Verwendung nach einem der vorhergehenden Ansprüche, bei der der Rohling mindestens ein Haltemittel zum Einspannen des Rohlings bei seiner Bearbeitung zu einem dentalen Formteil aufweist, wobei es sich bei dem Haltemittel vorzugsweise um ein am Außenumfang des Rohlings vorgesehenes Haltemittel handelt.

9. Verfahren zur Herstellung des in einem der Ansprüche 1 bis 8 definierten Rohlings, bei dem Glaspulver bei einem Druck zwischen 10 und 300 MPa, vorzugsweise zwischen 100 und 200 MPa, zu einem Grünkörper verpresst und dieser Grünkörper bei einer Temperatur zwischen 580 °C und 750 °C, vorzugsweise zwischen 620 °C und 660 °C, zu einem Rohling aus porösem Glas ohne kristalline Anteile gesintert, insbesondere vorgesintert, wird.

10. Verfahren zur Herstellung eines monolithischen dentalen Formteils, bei dem der in einem der Ansprüche 1 bis 8 definierte Rohling mechanisch bearbeitet wird, vorzugsweise durch mindestens einen Fräsvorgang, und das so erhaltene Formteil auf mindestens 98 % seiner theoretischen Dichte gesintert, insbesondere endgesintert, wird,
wobei das Formteil während mindestens eines Zeitabschnitts innerhalb der Sinterdauer mit mindestens einer Stützkonstruktion oder mit mindestens einer Stützform in Verbindung gebracht wird, derart, dass die Formänderung des Formteils während dieses Zeitabschnitts durch die Stützkonstruktion oder die Stützform begrenzt wird.

11. Verfahren nach Anspruch 10, bei dem ein solcher Zeitabschnitt mindestens am Ende der Sinterdauer vorgesehen ist.

12. Verfahren nach Anspruch 10 oder 11, bei dem die Stützkonstruktion oder die Stützform aus einem temperaturbeständigen Material besteht, das während des Sinterns seine Form um weniger als 2 Vol.-%, vorzugsweise um weniger als 1 Vol.-%, ändert.

13. Verfahren nach Anspruch 10 oder 11, bei dem die Stützkonstruktion oder die Stützform aus einem Material besteht, dessen Sinterschrumpf dem Sinterschrumpf des dentalen Formteils während des Sinterns entspricht.

14. Verfahren nach einem der Ansprüche 10 bis 13, bei dem das monolithische Formteil nach dem Sintern, insbesondere nach dem Endsintern, aus Glas, welches im Wesentlichen keine kristallinen Anteile besitzt, besteht.

15. Verfahren nach einem der Ansprüche 10 bis 14, bei dem die Sintertemperatur zwischen 800 °C und 1100 °C, vorzugsweise zwischen 900 °C und 1050 °C, beträgt.

16. Verfahren nach einem der Ansprüche 10 bis 15, bei dem das monolithische dentale Formteil einen Wärmeausdehnungskoeffizienten, gemessen nach ISO 6872, von weniger als 10 x 10⁻⁶ 1/K, vorzugsweise zwischen 7 und 10 x 10⁻⁶ 1/K, aufweist.

## Claims

1. Use of a blank, which entirely consists of porous glass without crystalline portions, wherein the density of this porous blank is between 50% and 95% of the theoretical density of a densely-sintered blank, and which has a disc-like shape with a diameter of at least 20 mm, for producing dental shaped parts.

2. Use according to claim 1, wherein the density of the blank is between 55% and 85%, preferably between 70% and 80%, of the theoretical density of the blank.

3. Use according to claim 1 or 2, wherein the glass consists of the following components:
| | |
|---|---|
| SiO₂ | 50 - 80 wt.-% |
| Al₂O₃ | 3 - 24 wt.-% |
| K₂O | 3 - 13 wt.-% |
| Na₂O | 3 - 13 wt.-% |
| Li₂O | 0 - 4 wt.-% |
| B₂O₃ | 0 - 4 wt.-% |
| F | 0 - 4 wt.-% |
| TiO₂ | 0 - 8 wt.-% |
| ZrO₂ | 0 - 8 wt.-% |
| P₂O₅ | 0 - 4 wt.-% |
| SnO₂ | 0 - 4 wt.-% |
| MgO | 0 - 4 wt.-% |
| CaO | 0 - 4 wt.-% |
| BaO | 0 - 4 wt.-% |
| Sb₂O₃ | 0 - 4 wt.-% |
| CeO₂ | 0 - 8 wt.-% |
and optionally coloring additives.

4. Use according to claim 3, wherein the glass has the following composition:
| | |
|---|---|
| SiO₂ | 55 - 65 wt.-% |
| Al₂O₃ | 17 - 24 wt.-% |
| K₂O | 5 - 9 wt.-% |
| Na₂O | 7 - 11 wt.-% |
| Li₂O | 0 - 1 wt.-% |
| B₂O₃ | 0 - 2 wt.-% |
| F | 0 - 1 wt.-% |
| TiO₂ | 0 - 1 wt.-% |
| ZrO₂ | 0 - 2 wt.-% |
| P₂O₅ | 0 - 1 wt.-% |
| SnO₂ | 0 - 1 wt.-% |
and optionally coloring additives.

5. Use according to claim 3, wherein the glass has the following composition:
| | |
|---|---|
| SiO₂ | 55 - 80 wt.-% |
| Al₂O₃ | 7 - 23 wt.-% |
| K₂O | 3 - 10 wt.-% |
| Na₂O | 3 - 13 wt.-% |
| Li₂O | 0 - 1 wt.-% |
| B₂O₃ | 0 - 4 wt.-% |
| F | 0 - 1 wt.-% |
| TiO₂ | 0 - 1 wt.-% |
| ZrO₂ | 0 - 8 wt.-% |
| P₂O₅ | 0 - 1 wt.-% |
and optionally coloring additives.

6. Use according to any one of the previous claims, wherein the blank has the shape of a substantially circular disk with a diameter of at least 20 mm, in particular of at least 50 mm, with diameters of at least 80 mm being further preferred.

7. Use according to any one of the previous claims, wherein the thickness of the disk is more than 5 mm, preferably between 5 mm and 30 mm.

8. Use according to any one of the previous claims, wherein the blank has at least one holding means for clamping the blank during the processing thereof to form a dental shaped part, and wherein the holding means is preferably a holding means provided on the outer circumference of the blank.

9. Process for producing the blank defined in any one of claims 1 to 8, wherein glass powder is pressed at a pressure of between 10 and 300 MPa, preferably between 100 and 200 MPa, to form a green body and this green body is sintered, in particular pre-sintered, at a temperature between 580°C and 750°C, preferably between 620°C and 660°C, to form a blank of porous glass without crystalline portions.

10. Process for producing a monolithic dental shaped part, wherein the blank defined in any one of claims 1 to 8 is machined, preferably by at least one milling step, and the thus-obtained shaped part is sintered, in particular finally sintered, to at least 98% of its theoretical density and wherein the shaped part is connected to at least one support structure or to at least one support mould during at least one time segment within the duration of the sintering, such that the change in shape of the shaped part during this time segment is limited by the support structure or the support mould.

11. Process according to claim 10, wherein such a time segment is provided at least at the end of the duration of the sintering.

12. Process according to claim 10 or claim 11, wherein the support structure or the support mould consists of a temperature-resistant material the shape of which changes during the sintering by less than 2 vol.-%, preferably by less than 1 vol.-%.

13. Process according to claim 10 or claim 11, wherein the support structure or the support mould consists of a material the sintering shrinkage of which corresponds to the sintering shrinkage of the dental shaped part during the sintering.

14. Process according to any one of claims 10 to 13, wherein the monolithic shaped part after the sintering, in particular after the final sintering, consists of glass which has essentially no crystalline portions.

15. Process according to any one of claims 10 to 14, wherein the sintering temperature is between 800°C and 1100°C, preferably between 900°C and 1050°C.

16. Process according to any one of claims 10 to 15, wherein the monolithic dental shaped part has a coefficient of thermal expansion, measured according to ISO 6872, of less than 10 x 10⁻⁶ 1/K, preferably of between 7 and 10 x 10⁻⁶ 1/K.

## Revendications

1. Utilisation d'une ébauche qui est constituée entièrement de verre poreux sans proportions cristallines, la densité de cette ébauche poreuse étant comprise entre 50 % et 95 % de la densité théorique d'une ébauche frittée à densité maximale, et qui présente la forme d'un disque d'un diamètre d'au moins 20 mm, en vue de l'élaboration de pièces moulées dentaires.

2. Utilisation selon la revendication 1, lors de laquelle la densité de l'ébauche est comprise entre 55 % et 85 %, de préférence entre 70 % et 80 %, de la densité théorique de l'ébauche.

3. Utilisation selon la revendication 1 ou 2, lors de laquelle le verre est constitué des composants suivants :
| | |
|---|---|
| SiO₂ | 50 à 80 % en poids |
| Al₂O₃ | 3 à 24 % en poids |
| K₂O | 3 à 13 % en poids |
| Na₂O | 3 à 13 % en poids |
| Li₂O | 0 à 4 % en poids |
| B₂O₃ | 0 à 4 % en poids |
| F | 0 à 4 % en poids |
| TiO₂ | 0 à 8 % en poids |
| ZrO₂ | 0 à 8 % en poids |
| P₂O₅ | 0 à 4 % en poids |
| SnO₂ | 0 à 4 % en poids |
| MgO | 0 à 4 % en poids |
| CaO | 0 à 4 % en poids |
| BaO | 0 à 4 % en poids |
| Sb₂O₃ | 0 à 4 % en poids |
| CeO₂ | 0 à 8 % en poids |
et, le cas échéant, des additifs colorants.

4. Utilisation selon la revendication 3, lors de laquelle le verre présente la composition suivante :
| | |
|---|---|
| SiO₂ | 55 à 65 % en poids |
| Al₂O₃ | 17 à 24 % en poids |
| K₂O | 5 à 9 % en poids |
| Na₂O | 7 à 11 % en poids |
| Li₂O | 0 à 1 % en poids |
| B₂O₃ | 0 à 2 % en poids |
| F | 0 à 1 % en poids |
| TiO₂ | 0 à 1 % en poids |
| ZrO₂ | 0 à 2 % en poids |
| P₂O₅ | 0 à 1 % en poids |
| SnO₂ | 0 à 1 % en poids |
et, le cas échéant, des additifs colorants.

5. Utilisation selon la revendication 3, lors de laquelle le verre présente la composition suivante :
| | |
|---|---|
| SiO₂ | 55 à 80 % en poids |
| Al₂O₃ | 7 à 23 % en poids |
| K₂O | 3 à 10 % en poids |
| Na₂O | 3 à 13 % en poids |
| Li₂O | 0 à 1 % en poids |
| B₂O₃ | 0 à 4 % en poids |
| F | 0 à 1 % en poids |
| TiO₂ | 0 à 1 % en poids |
| ZrO₂ | 0 à 8 % en poids |
| P₂O₅ | 0 à 1 % en poids |
et, le cas échéant, des additifs colorants.

6. Utilisation selon une des revendications précédentes, lors de laquelle l'ébauche présente la forme d'un disque sensiblement circulaire d'un diamètre d'au moins 20 mm, notamment d'au moins 50 mm, des diamètres d'au moins 80 mm étant particulièrement préférés.

7. Utilisation selon une des revendications précédentes, lors de laquelle l'épaisseur du disque est supérieure à 5 mm et est de préférence comprise entre 5 mm et 30 mm.

8. Utilisation selon une des revendications précédentes, lors de laquelle l'ébauche présente au moins un moyen de fixation destiné au serrage de l'ébauche lors de son élaboration pour obtenir une pièce moulée dentaire, le moyen de fixation étant de préférence un moyen de fixation prévu sur le pourtour extérieur de l'ébauche.

9. Procédé d'élaboration de l'ébauche définie dans une des revendications 1 à 8, selon lequel on comprime de la poudre de verre à une pression comprise entre 10 et 300 MPa, de préférence entre 100 et 200 MPa, pour obtenir un corps cru, et on soumet ce corps cru à un frittage, en particulier un préfrittage, à une température comprise entre 580 °C et 750 °C, de préférence entre 620 °C et 660 °C, pour obtenir une ébauche en verre poreux sans proportions cristallines.

10. Procédé d'élaboration d'une pièce moulée dentaire monolithique, selon lequel on soumet à un usinage mécanique l'ébauche définie dans l'une des revendications 1 à 8, de préférence lors d'au moins une opération de fraisage, et on soumet la pièce moulée dentaire ainsi obtenue à un frittage, notamment un frittage final, jusqu'à au moins 98 % de sa densité théorique, la pièce moulée étant reliée, pendant au moins un intervalle de temps au cours de la durée de frittage, à au moins une structure de support ou à au moins un moule de support, de telle sorte que pendant cet intervalle de temps, la variation de forme de la pièce moulée soit limitée par la structure de support ou le moule de support.

11. Procédé selon la revendication 10, selon lequel un tel intervalle de temps est prévu au moins à la fin de la durée de frittage.

12. Procédé selon la revendication 10 ou 11, selon lequel la structure de support ou le moule de support est constitué(e) d'un matériau résistant à la température, dont la forme varie de moins de 2 % en volume, de préférence de moins de 1 % en volume, au cours du frittage.

13. Procédé selon la revendication 10 ou 11, selon lequel la structure de support ou le moule de support est constitué(e) d'un matériau dont le retrait au frittage correspond au retrait au frittage de la pièce moulée dentaire au cours du frittage.

14. Procédé selon une des revendications 10 à 13, selon lequel, après le frittage, notamment après le frittage final, la pièce moulée monolithique est constituée d'un verre qui ne comporte sensiblement pas de proportions cristallines.

15. Procédé selon une des revendications 10 à 14, selon lequel la température de frittage est comprise entre 800 °C et 1 100 °C, de préférence entre 900 °C et 1 050 °C.

16. Procédé selon une des revendications 10 à 15, selon lequel la pièce moulée dentaire monolithique présente un coefficient de dilatation thermique, mesuré selon ISO 6872, de moins de 10 x 10⁻⁶ 1/K, de préférence entre 7 et 10 x 10⁻⁶ 1/K.
